# EUROPÄISCHE PATENTSCHRIFT

(11) **EP 3 089 778 B1**
(45) Veröffentlichungstag und Bekanntmachung des Hinweises auf die Patenterteilung: **26.06.2019**
(21) Anmeldenummer: 14835661.1
(22) Anmeldetag: 30.12.2014
(51) Int. Cl.: A61L 29/02, A61L 29/04, A61M 25/09, A61M 25/00, A61M 25/10

(54) **BALLONKATHETER**
BALLOON CATHETER
CATHÉTER À BALLONNET

(30) Priorität: 30.12.2013 DE 102013021998
(43) Veröffentlichungstag der Anmeldung: 09.11.2016
(73) Patentinhaber: Rübben, Alexander, 98000 Monaco (MC); Aachen Scientific International PTE. LTD., 079903 Singapore (SG)
(72) Erfinder: Rübben Alexander, 98000 Monaco (MC)
(74) Vertreter: Schneiders, Josef
(86) Internationale Anmeldenummer: PCT/EP2014/079417
(87) Internationale Veröffentlichungsnummer: WO 2015/101617

(56) Entgegenhaltungen:
- US-A1- 2006 004 328
- US-A1- 2010 056 985
- US-A1- 2012 220 935
- US-A1- 2013 178 795

## Beschreibung

Die Erfindung betrifft einen Ballonkatheter mit einem äußeren Tubus, an dessen distalem Ende sich ein Ballon anschließt, der durch Zufuhr eines Fluids über den äußeren Tubus expandierbar ist, wobei sich durch den äußeren Tubus zumindest teilweise ein innerer Tubus erstreckt, der durch den Ballon hindurch verläuft und distal des Ballons endet, wobei der innere Tubus ein Lumen zur Aufnahme eines Führungsdrahts und der Ballonkatheter einen distalen und einen proximalen Abschnitt aufweist, wobei der äußere Tubus im proximalen Abschnitt aus Metall und im distalen Abschnitt aus einem Kunststoffmaterial gefertigt ist, sodass der distale Abschnitt flexibler ist als der proximale Abschnitt.

Die sogenannten "minimalinvasiven Verfahren" nehmen in der Medizin einen immer größeren Stellenwert ein. Zur Behandlung von Gefäßverengungen wie Arteriosklerose wird häufig die perkutane transluminale Angioplastie (PTA) mittels Ballondilatation eingesetzt. Hierbei wird ein Ballonkatheter, der im distalen Bereich ein durch Fluidzufuhr expandierbares Element (Ballon) aufweist, mit Hilfe eines Führungskatheters zur Stenose (Gefäßverengung) gebracht. Der Ballonkatheter verfügt über ein Zufuhrlumen, das sich durch den Ballonkatheter bis proximal erstreckt und am distalen Ende mit dem Ballon in Verbindung steht. Durch Zufuhr eines Fluids wird der Ballon mit hohem Druck von in der Regel mindestens 4 bar, häufig 8 bis 12 bar, expandiert, sodass Ablagerungen im Bereich der Läsion gegen die Innenwand der Arterienwand gepresst werden, um auf diese Weise die Stenose zu beseitigen und den Blutdurchfluss zu verbessern. Um eine erneute Gefäßverengung zu verhindern, wird darüber hinaus zum Teil ein Stent implantiert, der das Gefäß offenhalten soll. Nach Zusammenfalten des Ballons zu einem kleineren Querschnitt wird der Ballonkatheter zurückgezogen und aus dem Gefäßsystem entfernt, während ein evtl. implantierter Stent im Gefäßsystem verbleibt. Der Zugang zum Blutgefäßsystem des Patienten erfolgt über ein Einführgerät, meist in der Leistengegend über die Arteria femoralis.

Ein Katheter zur Behandlung von Stenosen muss beim Vorschieben über das Blutgefäßsystem verschiedene Voraussetzungen erfüllen. Dabei ist zu beachten, dass der Vorschub häufig über vergleichsweise lange Strecken erfolgt, wenn etwa ein Katheter in der Leistengegend eingeführt und bis in den intrakraniellen Bereich vorgeschoben werden muss. Aus diesem Grund ist eine gewisse Steifheit des Katheters erforderlich. Auf der anderen Seite kann es jedoch erforderlich sein, den Katheter in englumige Blutgefäße vorzuschieben, hier wäre daher, um das Blutgefäß nicht zu verletzen und einen problemlosen Vorschub möglich zu machen, ein möglichst weiches Kathetermaterial von Vorteil. Um diese, sich grundsätzlich einander entgegenstehenden Bedingungen erfüllen zu können, werden bereits Ballonkatheter verwendet, in denen der proximale Teil aus einem Metall, insbesondere Edelstahl gefertigt ist, während ein distaler Abschnitt aus einem Kunststoffmaterial, beispielsweise Nylon besteht. Der aus Edelstahl gefertigte Anteil des Katheters wird häufig auch als Hypotube bezeichnet. Auf diese Weise erhält man einen Katheter, der einerseits, insbesondere in seinem proximalen Abschnitt, relativ steif ist, sodass auch über Distanzen von mehr als 1 m ein Vorschub möglich ist. Gleichzeitig erlaubt die Steifigkeit des Hypotubes auch die Übertragung von Drehmomenten. Auf der anderen Seite ist der distale Abschnitt des Katheters, der typischerweise erheblich kürzer ist als der proximale metallische Abschnitt, aus einem vergleichsweise weichen Kunststoffmaterial gefertigt, sodass der Katheter gerade im distalen Bereich auch engen Windungen der Blutgefäße problemlos folgt. Zudem gewährt der distale Abschnitt aus einem Polymer auch einen Schutz gegen ein unerwünschtes Abknicken des Katheters. Eine Knickbildung, die aufgrund der schwierigen Anpassung eines rein metallischen Ballonkatheters auftreten kann, hat nämlich zur Folge, dass ein weiterer Vorschub praktisch unmöglich wird und der Katheter letztlich nicht mehr verwendbar ist. Darüber hinaus besteht in erheblichem Maße die Gefahr, die Innenwandung des Blutgefäßes in Folge von Knickbildung zu verletzen, insbesondere wenn sich der Katheter bei weiterem Vorschub an der Abknickstelle aufwindet.

Die Patentschrift US 2010/056985 A1 offenbart einen Ballonkatheter mit einem länglichen Schaft bestehend aus einer inneren Schaft und einem äußeren Schaft. Der längliche Schaft besteht aus einem proximalen Abschnitt, einem mittleren Abschnitt und einem distalen Abschnitt. Der proximale Abschnitt ist beispielsweise aus Metall. Für den mittleren und distalen Abschnitt wird eine gleiche Vielzahl von Werkstoffen angegeben.

Die beschriebenen Maßnahmen haben zwar bereits dazu geführt, dass Ballonkatheter aus dem Stand der Technik einen vergleichsweise hohen Standard erreicht haben, dennoch stellt sich die Aufgabe, Ballonkatheter zur Verfügung zu stellen, die sich einerseits auch über lange Distanzen vorschieben lassen, andererseits aber auch den engen Windungen der Blutgefäße problemlos folgen.

Die Aufgabe wird erfindungsgemäß gelöst durch einen Ballonkatheter mit einem äußeren Tubus, an dessen distalem Ende sich ein Ballon anschließt, der durch Zufuhr eines Fluids über den äußeren Tubus expandierbar ist, wobei sich durch den äußeren Tubus zumindest teilweise ein innerer Tubus erstreckt, der durch den Ballon hindurch verläuft und distal des Ballons endet, wobei der innere Tubus ein Lumen zur Aufnahme eines Führungsdrahts und der Ballonkatheter einen distalen und einen proximalen Abschnitt aufweist, wobei der äußere Tubus im proximalen Abschnitt aus Metall und im distalen Abschnitt aus einem Kunststoffmaterial gefertigt ist, sodass der distale Abschnitt flexibler ist als der proximale Abschnitt, und wobei der distale Abschnitt sich aus einem ersten distalen Abschnitt und einem zweiten distalen Abschnitt zusammensetzt, der proximal des ersten distalen Abschnitts angeordnet ist, und der erste distale

Abschnitt flexibler ausgestaltet ist als der zweite distale Abschnitt, der äußere Tubus im ersten distalen Abschnitt aus einem weicheren Kunststoffmaterial gefertigt ist als im zweiten distalen Abschnitt und der innere Tubus im ersten distalen Abschnitt flexibler ausgestaltet ist als im zweiten distalen Abschnitt, wobei der innere Tubus im ersten und zweiten distalen Abschnitt jeweils aus dem gleichen Material gefertigt ist wie der äußere Tubus und der äußere Tubus und der innere Tubus im ersten distalen Abschnitt aus einem Polyetherblockamid und der äußere Tubus und der innere Tubus im zweiten distalen Abschnitt sowie der Ballon aus Polyhexamethylenadipinsäureamid gefertigt sind.

Erfindungsgemäß wird somit der distale Abschnitt des Ballonkatheters wiederum aufgeteilt in einen ersten und einen zweiten distalen Abschnitt. Dabei liegt der erste distale Abschnitt weiter distal als der zweite distale Abschnitt, d. h. der zweite distale Abschnitt schließt sich distal des proximalen Abschnitts aus Metall an, während der erste distale Abschnitt die am weitesten distal liegenden Bereiche des Ballonkatheters einschließlich des Ballons selbst umfasst.

Dadurch, dass eine zusätzliche Abstufung hinsichtlich der Weichheit bzw. Flexibilität des distalen Katheterabschnitts vorgenommen wird, verbessert sich die Einführbarkeit weiter, d. h. der erste distale Abschnitt sorgt dafür, dass der Teil des Ballonkatheters, der in die engsten Blutgefäße vordringen muss, besonders weich ist. Für die gesamte Länge des distalen Abschnitts wäre ein solch weiches Material jedoch eher ungeeignet, da ansonsten die Vorschiebbarkeit über größere Distanzen nicht mehr gewährleistet wäre. Aus diesem Grund wird ein zweiter distaler Abschnitt vorgesehen, der zwischen erstem distalen Abschnitt und dem proximalen Abschnitt gelegen ist und hinsichtlich Weichheit/Flexibilität zwischen dem metallischen proximalen Abschnitt und dem besonders weichen ersten distalen Abschnitt liegt. Unter Weichheit wird erfindungsgemäß die Flexibilität des jeweiligen Ballonkatheterabschnitts verstanden, d. h. die Fähigkeit, sich an die äußeren Gegebenheiten anzupassen. Die Begriffe "weich" und "flexibel" werden insofern synonym gebraucht.

Die unterschiedliche Weichheit von erstem und zweitem distalen Abschnitt lässt sich dadurch herbeiführen, dass man unterschiedliche Kunststoffmaterialien verwendet. Dabei ist das Kunststoffmaterial im ersten distalen Abschnitt weicher als im zweiten distalen Abschnitt.

Grundsätzlich sind unterschiedliche Kunststoffe bzw. Polymere für Ballonkatheter einsetzbar, beispielsweise Polyethylen, Polyurethan, Polyvinylchlorid, Polyamide, Polyimide, Silikone, Polyetheramide, Polytetraflurethylen oder EPDM (Ethylen-Propylen-Dien-Kautschuk).

Erfindungsgemäß finden für den ersten distalen Abschnitt Polyetherblockamide (PEBA) Verwendung. Hierbei handelt es sich um ein thermoplastisches Elastomer, das durch Polykondensation eines Carbonsäurepolyamids mit einem Polyether mit endständigen OH-Gruppen erhältlich ist. Insbesondere wird PEBA unter der Bezeichnung PEBAX® von der Firma Arkema vertrieben.

Alternativ, jedoch nicht unter die Erfindung fallend, können auch andere Polyamide für den ersten distalen Abschnitt Verwendung finden, insbesondere solche, wie sie unter Bezeichnung Grilamid® von der Firma EMS-GRIVORY vertrieben werden. Besonders bevorzugt ist die Verwendung eines Polyamids 12 (PA 12, Grilamid® L), einem durch die Polykondensation von Laurinlactam erhältlichen Polyamid. Weitere verwendbare Polyamide sind Polyamid 10.10 (PA 10.10, Grilamid® 1S), ein durch Polykondensation von Decandiamin und Sebacinsäure erhältliches Polyamid, Polyamid 6.10 (PA 6.10, Grilamid® 2S), ein durch Polykondensation von Hexamethylendiamin und Sebacinsäure erhältliches Polyamid oder Polyamid 6.12 (PA 6.12, Grilamid® 2D), ein durch Polykondensation von Hexamethylendiamin und Dodecandisäure erhältliches Polyamid.

Für den zweiten distalen Abschnitt wird erfindungsgemäß Polyhexamethylenadipinsäureamid (Nylon) verwendet. Dieses Material ist immer noch recht weich, wenn auch nicht so weich wie das für den ersten distalen Abschnitt verwendete Material, bspw. PEBA. Insbesondere kann für den ersten distalen Abschnitt ein Material mit einer Shore D-Härte im Bereich von ca. 25 - 72 verwendet werden. Für den zweiten distalen Abschnitt bietet sich hingegen die Verwendung von Materialien mit einer Shore D-Härte im Bereich von 80 - 85 an.

Sowohl hinsichtlich des ersten als auch hinsichtlich des zweiten distalen Abschnitts können die genauen Eigenschaften der Polymere durch Zugabe von Additiven, Weichmachern, Füllstoffen, Modifikatoren und Verarbeitungshilfsmittel eingestellt werden. Die entsprechenden Zusatzstoffe sind dem Fachmann grundsätzlich bekannt.

Sinnvollerweise ist, damit die Vorteile der unterschiedlichen Materialien sich insgesamt auf den Ballonkatheter auswirken, auch jeweils der innere Tubus im ersten distalen Abschnitt weicher ausgestaltet als im zweiten distalen Abschnitt. Erfindungsgemäß werden innerer und äußerer Tubus jeweils aus dem gleichen Material gefertigt. Auf diese Weise wird sichergestellt, dass der Ballonkatheter insgesamt im ersten distalen Abschnitt deutlich flexibler und besser anpassbar an die Innenwandungen der Blutgefäße ist als im zweiten distalen Abschnitt.

Zum ersten distalen Abschnitt ist zu beachten, dass zu diesem auch der Ballon selbst gehört, der jedoch typischerweise aus einem anderen Material gefertigt ist als innerer und äußerer Tubus im Bereich des ersten distalen Abschnitts, nämlich aus Polyhexamethylenadipinsäureamid (Nylon), das sich für die Fertigung von Ballonkathetern bewährt hat. Der äußere Tubus hingegen, der sich noch distal bis zum Ballon erstreckt und dann in diesen übergeht, ist im ersten distalen Abschnitt aus dem weicheren Material gefertigt, gleiches gilt auch für den inneren Tubus im ersten distalen Abschnitt, der sich durch den Ballon selbst hindurch bis distal des Ballons erstreckt.

Unter Ballon im Sinne der Erfindung wird das durch Zufuhr eines Fluids expandierbare Element eines Ballonkatheters verstanden, unabhängig davon, welche Form das expandierbare Element aufweist oder aus welchem Material es besteht. Der Ballonkatheter ist hinsichtlich seiner Dimensionen auf die Einführung in ein Körperlumen, insbesondere ein (Blut)gefäßsystem, abgestimmt. Dabei können die exakten Dimensionen variieren, je nachdem, ob das Blutgefäß bspw. eine Koronararterie, ein intrakranielles Blutgefäß oder eine Unterschenkelarterie ist.

Durch den inneren Tubus verläuft normalerweise ein Führungsdraht, der am distalen Ende des Ballonkatheters aus dem inneren Tubus austritt. Grundsätzlich sind verschiedene Ballonkatheter bekannt, insbesondere wird zwischen der Over-the-Wire-Technik (OTW-Katheter) und dem Rapid Exchange (Rx-Katheter) unterschieden. Während beim OTW-Katheter sich der Führungsdraht durch das Gesamtvolumen hindurch erstreckt, weisen Rx-Katheter eine Durchtrittsöffnung als Zugang zum inneren Tubus auf, durch die der Führungsdraht proximal des Ballons, aber distal des metallischen Hypotubes austreten kann. Vorteilhafterweise handelt es sich bei dem erfindungsgemäßen Ballonkatheter um einen Rx-Katheter, bei dem sich die Durchtrittsöffnung für den Führungsdraht im zweiten distalen Abschnitt befindet. Der äußere Tubus weist hier entsprechend eine Durchtrittsöffnung auf, die als Zugang zum inneren Tubus dient, d.h. der innere Tubus endet an dieser Stelle, die auch als Port bezeichnet wird.

Ein Vorteil eines Rx-Katheters liegt darin, dass aufgrund des verkürzten Führungsdrahtlumens, das durch den inneren Tubus gebildet wird, die Austauschbarkeit des Katheters über den Führungsdraht erleichtert wird. So kann die proximale Spitze des Führungsdrahts in die distale Öffnung des Führungsdrahtlumens im Ballonkatheter eingeführt werden, woraufhin der Katheter vorgeschoben wird, bis die proximale Spitze des Führungsdrahts durch den Port des Ballonkatheters wieder austritt. Der behandelnde Arzt kann somit sowohl den Führungsdraht als auch den Ballonkatheter ergreifen und den Ballonkatheter entlang des Führungsdrahts an seinen Bestimmungsort bringen. Bei einem OTW-Katheter hingegen müsste der Führungsdraht hierfür eine beträchtliche Länge aufweisen, was gleichzeitig mit Sterilisationsproblemen verbunden ist. Allerdings ist das Vorschieben eines Rx-Katheters unter Umständen schwieriger, da im proximalen Bereich des Katheters keine Versteifung durch den innenliegenden Führungsdraht erfolgt. Dieses Problem wird erfindungsgemäß jedoch dadurch behoben, dass der proximale Abschnitt metallisch als Hypotube ausgeführt ist.

Ein weiterer Vorteil eines Rx-Katheters ist darin zu sehen, dass im proximalen Abschnitt des Ballonkatheters kein separates Führungsdrahtlumen vorhanden sein muss. Entsprechend ist der Aufbau des Ballonkatheters in diesem Bereich einfacher und es kann ein niedriger Querschnitt vorgesehen sein. Darüber hinaus werden die Reibungskräfte zwischen Führungsdraht und Führungsdrahtlumen, d.h. Innenwandung des inneren Tubus, verringert, da der Führungsdraht größtenteils parallel zum Ballonkatheter und nicht durch den Ballonkatheter hindurch geführt wird.

Während der innere Tubus das Führungsdrahtlumen bildet, dient der äußere Tubus insbesondere auch als Fluidzufuhrlumen, d.h. das Fluid, insbesondere ein Gas, zum Expandieren des Ballons wird durch das Innere des äußeren Tubus dem Ballon zugeführt. Dieses Fluidzufuhrlumen erstreckt sich ohne Unterbrechung vom proximalen Abschnitt über den zweiten distalen Abschnitt und den ersten distalen Abschnitt zum Ballon. Der proximale Abschnitt des äußeren Tubus ist insbesondere aus Edelstahl gefertigt.

Selbstverständlich müssen die einzelnen Abschnitte miteinander verbunden werden, insbesondere können die Übergänge vom ersten distalen zum zweiten distalen Abschnitt und vom zweiten distalen Abschnitt zum proximalen Abschnitt durch Verschweißen geschaffen werden. Mit anderen Worten werden die verschiedenen Abschnitte des äußeren Tubus an den Übergangsstellen miteinander verschweißt. Gleiches gilt für den inneren Tubus, wobei der innere Tubus bei einem Rx-Katheter, der erfindungsgemäß bevorzugt ist, vor dem proximalen Abschnitt endet, sodass für den inneren Tubus kein Übergang vom zweiten distalen Abschnitt zum proximalen Abschnitt geschaffen werden muss. Alternativ zum Verschweißen sind aber auch andere Verbindungstechniken denkbar, wie beispielsweise Verkleben. Ggf. können sich die für die unterschiedlichen Abschnitte verwendeten Materialien an den Übergangsstellen vom ersten zum zweiten distalen Abschnitt sowie vom zweiten distalen Abschnitt zum proximalen Abschnitt leicht überlappen, um eine sichere Verbindung herzustellen.

Der erfindungsgemäß Ballonkatheter weist typischerweise eine Gesamtlänge von mehr als 1 m, insbesondere mehr als 1,4 m auf. Bevorzugt ist eine Gesamtlänge von ca. 145 cm, sodass eine Einführung in der Leistenregion möglich ist und der Ballonkatheter an unterschiedlichste Stellen bis hin in den intrakraniellen Bereich vorgeschoben werden kann. Von der Gesamtlänge macht in der Regel der proximale Abschnitt den Löwenanteil aus, dieser kann nämlich selbst mehr als 1 m lang sein. Die Länge des ersten distalen Abschnitts beträgt hingegen bevorzugt 10 - 20 cm, insbesondere 14 - 15 cm, wobei als erster distaler Abschnitt der gesamte Bereich von der distalen Spitze des Ballonkatheters bis zum Übergang zum zweiten distalen Abschnitt verstanden wird. Letzterer weist typischerweise eine Länge von 10 - 30 cm, insbesondere 15 - 25 cm auf. Der Abstand der Durchtrittsöffnung im zweiten distalen Abschnitt für den inneren Tubus vom proximalen Abschnitt beträgt typischerweise ca. 20 - 40 mm, insbesondere ca. 30 mm.

Ein typischer Außendurchmesser des äußeren Tubus im ersten distalen Abschnitt beträgt 0,8 - 1,0 mm, insbesondere ca. 0,9 mm. Der Innendurchmesser liegt typischerweise in einem Bereich von 0,7 - 0,8 mm. Der Außendurchmesser des inneren Tubus kann beispielsweise 0,5-0,6 mm, der Innendurchmesser des inneren Tubus 0,4 - 0,5 mm betragen.

Im zweiten distalen Abschnitt ist der Außendurchmesser des äußeren Tubus in der Regel geringfügig höher als im ersten distalen Abschnitt und beträgt typischerweise 0,9 - 1,1 mm, insbesondere ca. 0,95 mm. Der Innendurchmesser des äußeren Tubus liegt im zweiten distalen Abschnitt zumeist bei 0,8 - 0,9 mm, während der innere Tubus Dimensionen aufweist, die weitgehend denen im ersten distalen Abschnitt entsprechen.

Bei dem Ballon des Ballonkatheters kann es sich um einen medikamentenbeschichteten oder unbeschichteten Ballon handeln. Medikamentenbeschichtete Ballons dienen der Verhinderung der Restenose, indem auf dem Ballon ein Medikament aufgebracht wird, das beim Aufweiten an die Gefäßinnenwand abgegeben wird. Bei dem verwendeten Wirkstoff handelt es sich insbesondere um ein Arzneimittel bzw. Medikament, das proliferationshemmend wirkt und das gefäßverengende Überwuchern der durch den Ballon erweiterten Stelle verhindert. Insbesondere kann der Wirkstoff ausgewählt sein aus: Tretinoin, Orphanrezeptoragonisten, Elafinderivate, Corticosteroide, Steroidhormone, Paclitaxel, Rapamycin, Tacrolimus, hydrophobe Proteine sowie zellproliferationsverändernde Substanzen. Es ist auch möglich, Gemische dieser Wirkstoffe zu verwenden. Darüber hinaus können auch Derivate der genannten Wirkstoffe eingesetzt werden, wobei unter Derivaten insbesondere Salze, Ester und Amide verstanden werden. Als Steroidhormone können beispielsweise Methylprednisolon, Dexamethason oder Östradiol verwendet werden. Besonders bevorzugt ist die Verwendung von Paclitaxel bzw. Paclitaxelderivaten. Zusätzlich kann ggf. eine Polysaccharidbeschichtung, insbesondere eine Dextranbeschichtung aufgebracht werden, wie dies in der WO 2012/072074 A1 beschrieben wird. Auf diese Weise wird erreicht, dass der Wirkstoff nach der Expansion des Ballons gut an der Gefäßinnenwand haftet, sodass teilweise selbst Monate nach der Behandlung noch signifikante Wirkstoffkonzentrationen nachgewiesen werden können, die eine Restenose wirkungsvoll verhindern.

Ebenfalls möglich und sinnvoll ist es, einen Wirkstoff durch mehrfaches Benetzen mit einer Wirkstofflösung auf den Ballon aufzubringen, wobei zum Teil unterschiedliche Lösungsmittel Verwendung finden. Auf diese Weise wird die Wirkstoffbeschichtung insgesamt spröder, was für einen erhöhten Wirkstoffabtrag sorgt, wie es in der WO 2010/009904 A2 offenbart wird. Gemäß einer weiteren Variante der Wirkstoffaufbringung wird die Oberfläche des Ballons mechanisch, thermisch oder chemisch strukturiert, sodass die Oberfläche vergrößert und mit Vertiefungen versehen wird, wobei die Tiefe bzw. Breite typischerweise 5 - 50 µm beträgt.

Entlang des Ballonkatheters können an verschiedenen Positionen röntgendichte Markierungen angebracht sein, die der Visualisierung des Katheters im Röntgenbild dienen. Insbesondere kann es sich dabei um Markierungen aus Platin oder einer Platinlegierung handeln.

Am proximalen Ende des Ballonkatheters befindet sich typischerweise ein Anschlussstück, das grundsätzlich konusförmig ausgebildet sein kann, beispielsweise als sog. Luer-Konus. An dieser Stelle wird zum Aufblasen des Ballons eine Vorrichtung zur Gaszufuhr angeschlossen. Der ganz oder teilweise durch den Ballonkatheter verlaufende Führungsdraht wird an seinem proximalen Ende typischerweise durch einen Torquer gehalten, was die Handhabung des in der Regel sehr dünnen Führungsdrahts erleichtert.

Im Rahmen der Beschreibung wird unter dem Begriff proximal dem behandelnden Arzt zugewandt verstanden, d. h. das proximale Ende weist in Richtung Körperäußeres. Umgekehrt bedeutet distal dem Arzt abgewandt, das distale Ende liegt also in Richtung Körperinneres.

Die Erfindung wird anhand der beigefügten Figur näher erläutert. Es zeigt:

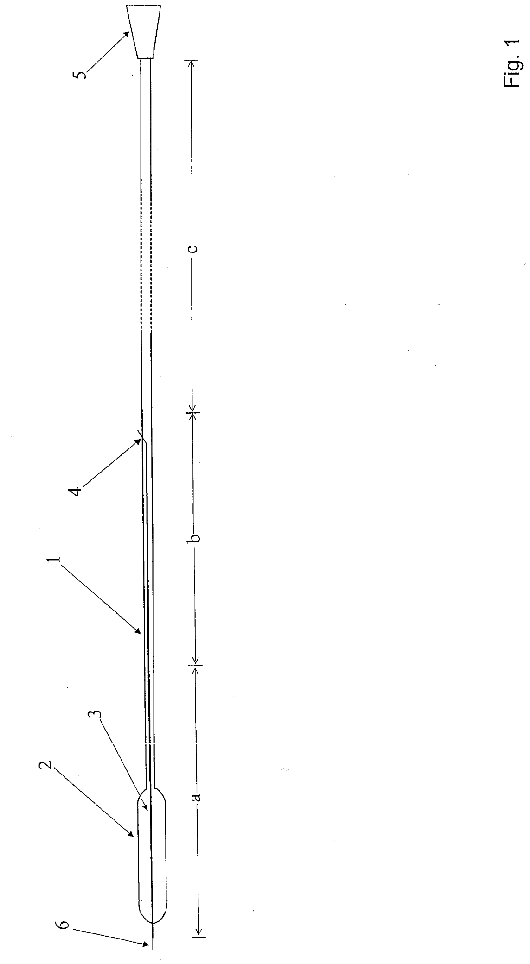

- Fig. 1:: Einen erfindungsgemäßen Ballonkatheter in der Seitenansicht.

Der erfindungsgemäße Ballonkatheter weist einen äußeren Tubus 1 auf, durch den abschnittsweise ein weiterer innerer Tubus 3 verläuft. Am proximalen Ende des Ballonkatheters befindet sich ein Anschlussstück 5, während das distale Ende des äußeren Tubus 1 in den Ballon 2 übergeht. Wenn der Ballon 2 expandiert werden soll, wird das Anschlussstück 5 an eine Vorrichtung zur Gaszufuhr angeschlossen und der Ballon 2 expandiert. Im Anschluss daran wird der Ballon 2 wieder komprimiert, indem das Fluid abgezogen wird. Das Fluidzufuhrlumen verläuft dabei durch den äußeren Tubus 1.

Durch den inneren Tubus 3 kann der Führungsdraht 6 geführt werden, der am distalen Ende des Ballonkatheters austritt. Bei einem Rx-Katheter weist der äußere Tubus 1 eine auch Port genannte Durchtrittsöffnung 4 auf, an der der innere Tubus 3 endet und durch die der Führungsdraht 6 proximal den Ballonkatheter verlässt. Von hier aus kann der Führungsdraht 6 außerhalb des Ballonkatheters in Richtung proximal weitergeführt und außerhalb des Körpers mithilfe eines Torquers vom behandelnden Arzt gefasst werden.

Der Ballonkatheter weist im Wesentlichen drei Abschnitte auf, einen ersten distalen Abschnitt a, einen zweiten distalen Abschnitt b und einen proximalen Abschnitt c. Diese sind unterschiedlich flexibel ausgestaltet, wobei in der Regel unterschiedliche Materialien zum Einsatz kommen. Der proximale Abschnitt c ist am längsten und aus einem Metall, insbesondere Edelstahl gefertigt. Hieran schließt sich der zweite distale Abschnitt b an, der aus einem weichen, aber nicht extrem weichen Polymermaterial, nämlich Polyhexamethylenadipinsäreamid gefertigt ist. Schließlich geht der zweite distale Abschnitt b weiter distal in den ersten distalen Abschnitt a über; dieser ist besonders flexibel, was dadurch erreicht wird, dass sowohl der innere als auch der äußere Tubus 1,3 aus einem besonders weichen Material, insbesondere PEBA gefertigt sind. Der Ballon 2 selbst hingegen besteht erfindungsgemäß aus Polyhexamethylenadipinsäreamid.

## Patentansprüche

1. Ballonkatheter mit einem äußeren Tubus (1), an dessen distalem Ende sich ein Ballon (2) anschließt, der durch Zufuhr eines Fluids über den äußeren Tubus (1) expandierbar ist, wobei sich durch den äußeren Tubus (1) zumindest teilweise ein innerer Tubus (3) erstreckt, der durch den Ballon (2) hindurch verläuft und distal des Ballons (2) endet, wobei der innere Tubus (3) ein Lumen zur Aufnahme eines Führungsdrahts (6) und der Ballonkatheter einen distalen (a,b) und einen proximalen Abschnitt (c) aufweist, wobei der äußere Tubus (1) im proximalen Abschnitt (c) aus Metall und im distalen Abschnitt (a,b) aus einem Kunststoffmaterial gefertigt ist, sodass der distale Abschnitt (a,b) flexibler ist als der proximale Abschnitt (c), und wobei der distale Abschnitt (a,b), sich aus einem ersten distalen Abschnitt (a) und einem zweiten distalen Abschnitt (b) zusammensetzt, der proximal des ersten distalen Abschnitts (a) angeordnet ist,
**dadurch gekennzeichnet, dass** der erste distale Abschnitt (a) flexibler ausgestaltet ist als der zweite distale Abschnitt (b), der äußere Tubus (1) im ersten distalen Abschnitt (a) aus einem weicheren Kunststoffmaterial gefertigt ist als im zweiten distalen Abschnitt (b) und der innere Tubus (3) im ersten distalen Abschnitt (a) flexibler ausgestaltet ist als im zweiten distalen Abschnitt (b), wobei der innere Tubus (3) im ersten und zweiten distalen Abschnitt (a,b) jeweils aus dem gleichen Material gefertigt ist wie der äußere Tubus (1) und der äußere Tubus (1) und der innere Tubus (3) im ersten distalen Abschnitt (a) aus einem Polyetherblockamid und der äußere Tubus (1) und der innere Tubus (3) im zweiten distalen Abschnitt (b) sowie der Ballon (2) aus Polyhexamethylenadipinsäureamid gefertigt sind.

2. Ballonkatheter nach Anspruch 1, **dadurch gekennzeichnet, dass** der äußere Tubus (1) im ersten distalen Abschnitt (a) aus einem Polyamid 12 gefertigt ist.

3. Ballonkatheter nach Anspruch 1 oder 2, **dadurch gekennzeichnet, dass** der äußere Tubus (1) im zweiten distalen Abschnitt (b) eine Durchtrittsöffnung (4) als Zugang zum inneren Tubus (3) aufweist.

4. Ballonkatheter nach einem der Ansprüche 1 bis 3, **dadurch gekennzeichnet, dass** der proximale Abschnitt (c) des äußeren Tubus (1) aus Edelstahl gefertigt ist.

5. Ballonkatheter nach einem der Ansprüche 1 bis 4, **gekennzeichnet durch** eine Gesamtlänge von mehr als 1 m, insbesondere mehr als 1,4 m.

6. Ballonkatheter nach einem der Ansprüche 1 bis 5, **dadurch gekennzeichnet, dass** die Länge des ersten distalen Abschnitts 10-20 cm, insbesondere 14 - 15 cm beträgt.

7. Ballonkatheter nach einem der Ansprüche 1 bis 6, **dadurch gekennzeichnet, dass** die Länge des zweiten distalen Abschnitts 10 - 30 cm, insbesondere 15 - 25 cm beträgt.

8. Ballonkatheter nach einem der Ansprüche 1 bis 7, **dadurch gekennzeichnet, dass** der Außendurchmesser des äußeren Tubus (1) im ersten distalen Abschnitt (a) 0,8 - 1,0 mm beträgt.

9. Ballonkatheter nach einem der Ansprüche 1 bis 8, **dadurch gekennzeichnet, dass** der Außendurchmesser des äußeren Tubus (1) im zweiten distalen Abschnitt (b) 0,9 - 1,1 mm beträgt.

## Claims

1. Balloon catheter comprising an outer tube (1) to which distal end a balloon (2) connects that can be expanded by feeding in a fluid via the outer tube (1), wherein an inner tube (3) extends at least partially through the outer tube (1), said inner tube (3) passing through the balloon (2) and terminating distally of the balloon (2), with the inner tube (3) having a lumen appropriate to accommodate a guidewire (6) and the balloon catheter having a distal (a,b) and a proximal section (c), wherein the proximal section (3) of the outer tube (1) is made of metal and the distal section (a,b) is made of plastic material making the distal section (a,b) more flexible than the proximal section (c), the distal section (a,b) comprises a first distal section (a) and a second distal section (b), said second distal section (b) being located proximal to the first distal section (a),
**characterized in that** the first distal section (a) being made so as to be more flexible than the second distal section (b), the outer tube (1) in the first distal section (a) is made of a softer plastic material than in the second distal section (b), the inner tube (3) in the first distal section (a) is more flexible than in the second distal section (b), wherein the inner tube (3) of the first and second distal section (a, b) is each made of the same material as the outer tube (1) and the outer tube (1) and the inner tube (3) in the first distal section (a) are made of a polyether block amide, and the outer tube (1) and the inner tube (3) in the second distal section (b) as well as the balloon (2) are made of polyhexamethylene adipic acid amide.

2. Balloon catheter according to claim 1, **characterized in that** the outer tube (1) in the first distal section (a) is made of a polyamide 12.

3. Balloon catheter according to claim 1 or 2, **characterized in that** the outer tube (1) in the second distal section (b) is provided with a passage opening (4) allowing access to the inner tube (3).

4. Balloon catheter according to any of claims 1 to 3, **characterized in that** the proximal section (c) of the outer tube (1) is made of stainless steel.

5. Balloon catheter according to any of claims 1 to 4, **characterized by** a total length of more than 1 m, in particular more than 1.4 m.

6. Balloon catheter according to any of claims 1 to 5, **characterized in that** the length of the first distal section amounts to 10 to 20 cm, in particular is 14 to 15 cm.

7. Balloon catheter according to any of claims 1 to 6, **characterized in that** the length of the second distal section amounts to 10 to 30 cm, in particular 15 to 25 cm.

8. Balloon catheter according to any of claims 1 to 7, **characterized in that** the outside diameter of the outer tube (1) in the first distal section (a) amounts to 0.8 to 1.0 mm

9. Balloon catheter according to any of claims 1 to 8, **characterized in that** the outside diameter of the outer tube (1) in the second distal section (b) amounts to 0.9 to 1.1 mm.

## Revendications

1. Cathéter à ballonnet comprenant un tube extérieur (1) à l'extrémité distale duquel se raccorde un ballonnet (2) qui peut être gonflé par l'alimentation en fluide par le biais du tube extérieur (1), un tube intérieur (3) s'étendant au moins en partie à travers le tube extérieur (1), lequel s'étend à travers le ballonnet (2) et se termine distalement par rapport au ballonnet (2), le tube intérieur (3) présentant une lumière pour recevoir un fil de guidage (6) et le cathéter à ballonnet présentant une portion distale (a, b) et une portion proximale (c), le tube extérieur (1) dans la portion proximale (c) étant fabriqué en métal et dans la portion distale (a, b) étant fabriqué en une matière plastique de telle sorte que la portion distale (a, b) soit plus flexible que la portion proximale (c), et la portion distale (a, b) se composant d'une première portion distale (a) et d'une deuxième portion distale (b) qui est disposée proximalement par rapport à la première portion distale (a),
**caractérisé en ce que**
la première portion distale (a) est réalisée de manière plus flexible que la deuxième portion distale (b), le tube extérieur (1) est fabriqué dans la première portion distale (a) en une matière plastique plus tendre que dans la deuxième portion distale (b) et le tube intérieur (3) est configuré sous forme plus flexible dans la première portion distale (a) que dans la deuxième portion distale (b), le tube intérieur (3), dans la première et dans la deuxième portion distale (a, b), étant fabriqué à chaque fois en le même matériau que le tube extérieur (1) et le tube extérieur (1) et le tube intérieur (3), dans la première portion distale (a), étant fabriqués en un polyéther bloc amide et le tube extérieur (1) et le tube intérieur (3), dans la deuxième portion distale (b) ainsi que le ballonnet (2), étant fabriqués en polyhexaméthylèneadipamide.

2. Cathéter à ballonnet selon la revendication 1,
**caractérisé en ce que** le tube extérieur (1) est fabriqué dans la première portion distale (a) en un polyamide 12.

3. Cathéter à ballonnet selon la revendication 1 ou 2, **caractérisé en ce que** le tube extérieur (1) présente, dans la deuxième portion distale (b), une ouverture de passage (4) en tant qu'accès au tube intérieur (3).

4. Cathéter à ballonnet selon l'une quelconque des revendications 1 à 3, **caractérisé en ce que** la portion proximale (c) du tube extérieur (1) est fabriquée en acier inoxydable.

5. Cathéter à ballonnet selon l'une quelconque des revendications 1 à 4, **caractérisé par** une longueur totale supérieure à 1 m, en particulier supérieure à 1,4 m.

6. Cathéter à ballonnet selon l'une quelconque des revendications 1 à 5, **caractérisé en ce que** la longueur de la première portion distale mesure 10 à 20 cm, en particulier 14 à 15 cm.

7. Cathéter à ballonnet selon l'une quelconque des revendications 1 à 6, **caractérisé en ce que** la longueur de la deuxième portion distale mesure 10 à 30 cm, en particulier 15 à 25 cm.

8. Cathéter à ballonnet selon l'une quelconque des revendications 1 à 7, **caractérisé en ce que** le diamètre extérieur du tube extérieur (1) dans la première portion distale (a) mesure 0,8 à 1,0 mm.

9. Cathéter à ballonnet selon l'une quelconque des revendications 1 à 8, **caractérisé en ce que** le diamètre extérieur du tube extérieur (1) dans la deuxième portion distale (b) mesure 0,9 à 1,1 mm.
